# EUROPEAN PATENT APPLICATION

(11) **EP 4 769 423 A1**
(43) Date of publication of application: **01.07.2026**
(21) Application number: 25223848.0
(22) Date of filing: 16.12.2025
(51) Int. Cl.: G16H 20/40, A61N 5/10, G16H 30/40, G16H 50/30

(54) **MULTI-MODEL VALIDATION OF RADIOTHERAPY MACHINE LEARNING MODELS**

(30) Priority: 27.12.2024 US 202419003107
(71) Applicant: Siemens Healthineers International AG, 6312 Steinhausen (CH)
(72) Inventor: KUUSELA, Esa, 00100 Helsinki (FI)
(74) Representative: Mathisen & Macara LLP

(57) **Abstract**

Disclosed herein are methods (200) and systems (100) for optimizing radiation therapy plans through a dual-model system, enhancing operational efficiency, effectiveness, and safety of machine learning models (111, 112) configured to generate radiotherapy treatment plans. Embodiments utilize a utility function to guide the optimization of treatment parameters, while an independent validation model (111) evaluates each iteration of the plan. The validation model (111) ensures that the treatment remains clinically viable by halting the optimization process (200) once the validation score ceases to improve, thereby preventing over-optimization. This approach allows for optimized treatment plans to be predicted by existing machine learning in a manner that is both theoretically ideal but also practically applicable and safe for patient treatment, addressing the critical need for reliability in radiation therapy planning.

## Description

### TECHNICAL FIELD

This application relates generally to using a multi-model approach to validate and improve the operational efficiency of machine learning models predicting radiotherapy treatment attributes.

### BACKGROUND

Radiation therapy treatment planning (RTTP) is a complex process that contains specific guidelines, protocols, and instructions adopted by different medical professionals, such as clinicians, medical device manufacturers, treating physicians, and the like. Due to the extreme nature of radiation emitted from radiotherapy machines, it is imperative that all the instructions are precisely followed. Field geometry, as used in the context of RTTP, refers to various attributes or settings of a radiotherapy machine while a patient receives a prescribed radiotherapy dose. For instance, a prescribing physician may identify a source location (e.g., the patient's organ to be treated or tumor to be eradicated) and a corresponding dosage. Moreover, other parties (e.g., clinicians or machine manufacturers) may determine positioning attributes (e.g., angles) of the gantry and the patient on the couch to provide optimum treatment.

In the field of RTTP generation, optimization has evolved from a manual process where planners directly specified desired dose distributions, often using dose-volume histograms (DVH), to a more sophisticated model-based approach. The rise of automation and artificial intelligence techniques have allowed treatment planning to increasingly rely on predictive modeling paradigms, such as machine learning models. These machine learning models, which can range from simple outcome predictions based on DVH metrics to advanced systems, use (e.g., train on) historical data to predict the likely outcomes of an RTTP. The introduction of AI has further enhanced these models, allowing for predictions about tumor control and side effects based on machine control points, potentially leading to more personalized and effective treatments.

However, the reliance on empirical and/or AI models has also introduced a new set of technical challenges. For instance, conventional machine learning models analyzing radiotherapy treatment plans generally perform well only within the confines of the data they were trained on. As a result, even sophisticated machine learning models struggle when faced with unique cases that are not well-represented within the training dataset used to train those sophisticated models. For instance, when a plan optimizer fully utilizes the model's capabilities, it might create plans that differ from the original training examples, potentially leading to clinically unacceptable results. In some embodiments, the machine learning models may continue reiterating in order to converge upon the desired prediction, thereby using undesirable amounts of computing resources and further resulting in longer-than-expected run times. This issue is exacerbated when the optimization process is allowed to explore more complex solutions, such as adjusting control points or increasing modulation, as seen in techniques like VMAT optimization.

Therefore, existing and conventional models have proven to be operationally inefficient. Specifically, the computation or prediction time is longer than desired, and, in some cases, some machine learning models require high computing resources (e.g., GPUs or memory resources), which is also highly undesirable.

### SUMMARY

In accordance with a first aspect of the invention, there is provided a method as defined by claim 1.

In accordance with a second aspect of the invention, there is provided a computer system as defined by claim 8.

Optional features are defined by the dependent claims.

For the aforementioned reasons, there is a desire for a system that can utilize a computer model (e.g., an AI or machine-learning model) to improve the operational efficiency of the existing machine-learning models predicting radiotherapy treatment attributes (e.g., plan optimizers).

The methods and systems discussed herein address the above-identified technical challenges by introducing a validation model that can be executed alongside the conventional plan optimizer. The validation model can provide an independent check on the optimization process of a sophisticated machine learning model, such as the plan optimizer. This approach ensures that even as the plan optimizer explores complex solutions, the final treatment plans remain within clinically acceptable boundaries. This paradigm improves the RTTPs generated using traditional cost functions and enhances the reliability and effectiveness of the optimization process. Moreover, the methods and systems discussed herein allow for faster convergence of data, thereby achieving the same (or better) results than achieved via conventional machine learning models in a faster manner and/or using less computing resources.

Some entities have tried to modify or retrain their plan optimizers (machine learning models that predict radiotherapy models) in order to solve the technical problems discussed herein. However, modifying existing machine learning models to address the technical challenges discussed herein is also not desirable due to several factors. First, altering foundational model parameters or structures could inadvertently introduce new biases or reduce the generalizability of the models, especially when the modified model encounters patient data that were underrepresented in the training set. Moreover, significant changes to an existing model's architecture might necessitate extensive retraining, consuming valuable resources and time and potentially delaying clinical applications. Additionally, continuous adaptations might lead to a complex model management cycle where ensuring the consistency and predictability of treatment outcomes becomes challenging. In contrast, the validation model discussed herein may operate independently and may be used to determine when the optimization process should be terminated. Therefore, the validation model can be used as a retrofit measure and without disrupting a clinic's existing computing infrastructure. The methods and systems discussed herein can be used to supplement the existing computing infrastructure of a clinic (e.g., existing machine learning models) with a parallel validation system for maintaining the stability of the treatment planning process while enhancing its robustness and clinical relevance.

The methods and systems discussed herein address several technical shortcomings inherent in conventional model-based radiation therapy treatment planning. Conventional models often underperform when faced with patient cases that vary significantly from the training data set. To overcome this challenge, the methods and systems discussed herein incorporate a validation model that operates independently from the utility function used in the optimization process. The validation model can ensure robustness and broad applicability of treatment plans across diverse patient scenarios.

Another major challenge with existing optimization approaches is the risk of over-optimization, where conventional models generate treatment plans that are theoretically optimal according to model parameters but may not align with clinical effectiveness. Employing the dual-model approach discussed herein prevents the optimization from overly conforming to the model's specific features, thereby ensuring clinical viability and faster convergence time.

Traditional treatment planning methods often fail to adapt dynamically to unique, patient-specific factors during optimization. The methods and systems discussed herein address this issue by allowing for the dynamic adjustment of the optimization's stopping threshold based on real-time analysis of validation score changes or direct physician inputs. Such adaptability ensures that the treatment plans are personalized and optimized for individual patient conditions, enhancing both safety and efficacy.

Moreover, current optimization processes in radiation therapy planning can be labor-intensive and time-consuming, necessitating multiple iterations and adjustments. The methods and systems discussed herein improve operational efficiency by utilizing a validation score to determine the optimal stopping point for the optimization process. This adjustment potentially reduces the number of iterations required to reach a clinically acceptable plan, streamlining the planning process.

The validation model discussed herein can provide an additional layer of scrutiny, or an additional layer of plan assessment. Accordingly, the validation model evaluates the quality of the plan based on different criteria, and the optimization is stopped when the validation model's score ceases to improve or ceases to improve by a threshold, even if the utility function continues to show gains. This approach prevents the optimizer from overfitting or pushing the plan into clinically untested territories.

In some aspects, the techniques described herein relate to a method including: receiving, by at least one processor, an indication that a first computer model has initiated an iterative process of generating a radiation therapy treatment plan by ingesting a set of treatment plan attributes; retrieving, by at least one processor after a first iteration of the first computer model, a first set of treatment attributes associated with a first treatment plan generated during the first iteration; executing, by the at least one processor, a second computer model to generate a first validation score for the first treatment plan using the first set of treatment attributes; retrieving, by at least one processor after a second iteration of the first computer model, a second set of treatment attributes associated with a second treatment plan generated during the second iteration; executing, by the at least one processor, the second computer model to generate a second validation score for the second treatment plan using the second set of treatment attributes; calculating, by the at least one processor, a first radiotherapy utility improvement value corresponding to a difference between the first validation score and the second validation score; retrieving, by at least one processor after a third iteration of the first computer model, a third set of treatment attributes associated with a third treatment plan generated during the third iteration; executing, by the at least one processor, the second computer model to generate a third validation score for the third treatment plan using the third set of treatment attributes; calculating, by the at least one processor, a second radiotherapy utility improvement value corresponding to a difference between the second validation score and the third validation score; and when a difference between the first radiotherapy utility improvement value and the second radiotherapy utility improvement value satisfies a threshold, instructing, by the at least one processor, the first computer model to stop executing a future iteration.

In some aspects, the techniques described herein relate to a method, wherein the validation score corresponds to whether a treatment plan is within a clinical boundary.

In some aspects, the techniques described herein relate to a method, wherein the clinical boundary corresponds to an organ at risk dose limit, a beam angle constraint, a hot spot attribute, a cold spot attribute, and/or a planning target volume dose limit.

In some aspects, the techniques described herein relate to a method, wherein the second computer model is configured to generate the validation score based on a different set of criteria than those used by the first computer model, optionally the set of criteria including clinical guidelines, historical treatment outcomes, and/or predictive models for side effects. For example, the different set of criteria may include clinical guidelines, historical treatment outcomes, and/or predictive models for side effects.

In some aspects, the techniques described herein relate to a method, further including: adjusting, by at least one processor, the threshold used to determine when to stop the first computer model's iterative process based on a patient-specific risk profile and/or physician input.

In some aspects, the techniques described herein relate to a method, wherein the threshold is dynamically adjusted during the iterative process based on real-time analysis of a rate of change in any of the validation scores.

In some aspects, the techniques described herein relate to a method, further including: outputting, by the at least one processor, the third set of treatment attributes associated with a third treatment plan as a final result of execution of the first computer model.

In some aspects, the techniques described herein relate to a system including a computer-readable medium having non-transitory instructions, that when executed, cause a processor to: receive an indication that a first computer model has initiated an iterative process of generating a radiation therapy treatment plan by ingesting a set of treatment plan attributes; retrieve, after a first iteration of the first computer model, a first set of treatment attributes associated with a first treatment plan generated during the first iteration; execute a second computer model to generate a first validation score for the first treatment plan using the first set of treatment attributes; retrieve, after a second iteration of the first computer model, a second set of treatment attributes associated with a second treatment plan generated during the second iteration; execute the second computer model to generate a second validation score for the second treatment plan using the second set of treatment attributes; calculate a first radiotherapy utility improvement value corresponding to a difference between the first validation score and the second validation score; retrieve, after a third iteration of the first computer model, a third set of treatment attributes associated with a third treatment plan generated during the third iteration; execute the second computer model to generate a third validation score for the third treatment plan using the third set of treatment attributes; calculate a second radiotherapy utility improvement value corresponding to a difference between the second validation score and the third validation score; and when a difference between the first radiotherapy utility improvement value and the second radiotherapy utility improvement value satisfies a threshold, instruct the first computer model to stop executing a future iteration.

In some aspects, the techniques described herein relate to a system, wherein the validation score corresponds to whether a treatment plan is within a clinical boundary.

In some aspects, the techniques described herein relate to a system, wherein the clinical boundary corresponds to an organ at risk dose limit, a beam angle constraint, a hot spot attribute, a cold spot attribute, and/or a planning target volume dose limit.

In some aspects, the techniques described herein relate to a system, wherein the second computer model is configured to generate the validation score based on a different set of criteria than those used by the first computer model, optionally the set of criteria including clinical guidelines, historical treatment outcomes, and/or predictive models for side effects. For example, the different set of criteria may include clinical guidelines, historical treatment outcomes, and/or predictive models for side effects.

In some aspects, the techniques described herein relate to a system, wherein the instructions further cause the processor to: adjust the threshold used to determine when to stop the first computer model's iterative process based on a patient-specific risk profile and/or physician input.

In some aspects, the techniques described herein relate to a system, wherein the threshold is dynamically adjusted during the iterative process based on real-time analysis of a rate of change in any of the validation scores.

In some aspects, the techniques described herein relate to a system, wherein the instructions further cause the processor to: output the third set of treatment attributes associated with a third treatment plan as a final result of execution of the first computer model.

In some aspects, the techniques described herein relate to a system including: a first computer model configured to generate a radiation therapy treatment plan by ingesting a set of treatment plan attributes; a second computer model configured to validate the first computer model; and a processor in communication with the first computer model and the second computer model, the processor configured to: receive an indication that the first computer model has initiated an iterative process of generating the radiation therapy treatment plan by ingesting the set of treatment plan attributes; retrieve, after a first iteration of the first computer model, a first set of treatment attributes associated with a first treatment plan generated during the first iteration; execute the second computer model to generate a first validation score for the first treatment plan using the first set of treatment attributes; retrieve, after a second iteration of the first computer model, a second set of treatment attributes associated with a second treatment plan generated during the second iteration; execute the second computer model to generate a second validation score for the second treatment plan using the second set of treatment attributes; calculate a first radiotherapy utility improvement value corresponding to a difference between the first validation score and the second validation score; retrieve, after a third iteration of the first computer model, a third set of treatment attributes associated with a third treatment plan generated during the third iteration; execute the second computer model to generate a third validation score for the third treatment plan using the third set of treatment attributes; calculate a second radiotherapy utility improvement value corresponding to a difference between the second validation score and the third validation score; and when a difference between the first radiotherapy utility improvement value and the second radiotherapy utility improvement value satisfies a threshold, instruct the first computer model to stop executing a future iteration.

In some aspects, the techniques described herein relate to a system, wherein the validation score corresponds to whether a treatment plan is within a clinical boundary.

In some aspects, the techniques described herein relate to a system, wherein the clinical boundary corresponds to an organ at risk dose limit, a beam angle constraint, a hot spot attribute, a cold spot attribute, and/or a planning target volume dose limit.

In some aspects, the techniques described herein relate to a system, wherein the second computer model is configured to generate the validation score based on a different set of criteria than those used by the first computer model, optionally the set of criteria including clinical guidelines, historical treatment outcomes, and/or predictive models for side effects. For example, the different set of criteria may include clinical guidelines, historical treatment outcomes, and/or predictive models for side effects.

In some aspects, the techniques described herein relate to a system, wherein processor is further configured to adjust the threshold used to determine when to stop the first computer model's iterative process based on a patient-specific risk profile and/or physician input.

In some aspects, the techniques described herein relate to a system, wherein the threshold is dynamically adjusted during the iterative process based on real-time analysis of a rate of change in any of the validation scores.

### BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.
**FIG.** 1 illustrates components of a multi-model radiotherapy planning validation system, according to an embodiment.
**FIG.** 2 illustrates an example flow diagram of a multi-model radiotherapy planning validation system, according to an embodiment.
**FIG.** 3 illustrates an example flow diagram of a multi-model radiotherapy planning validation system, according to an embodiment.

### DETAILED DESCRIPTION

Reference will now be made to the illustrative embodiments depicted in the drawings, and specific language will be used here to describe the same. It will nevertheless be understood that no limitation of the scope of the claims or this disclosure is thereby intended. Alterations and further modifications of the inventive features illustrated herein, and additional applications of the principles of the subject matter illustrated herein, which would occur to one skilled in the relevant art and having possession of this disclosure, are to be considered within the scope of the subject matter disclosed herein. Other embodiments may be used and/or other changes may be made without departing from the scope of the present disclosure. The illustrative embodiments described in the detailed description are not meant to be limiting of the subject matter presented.

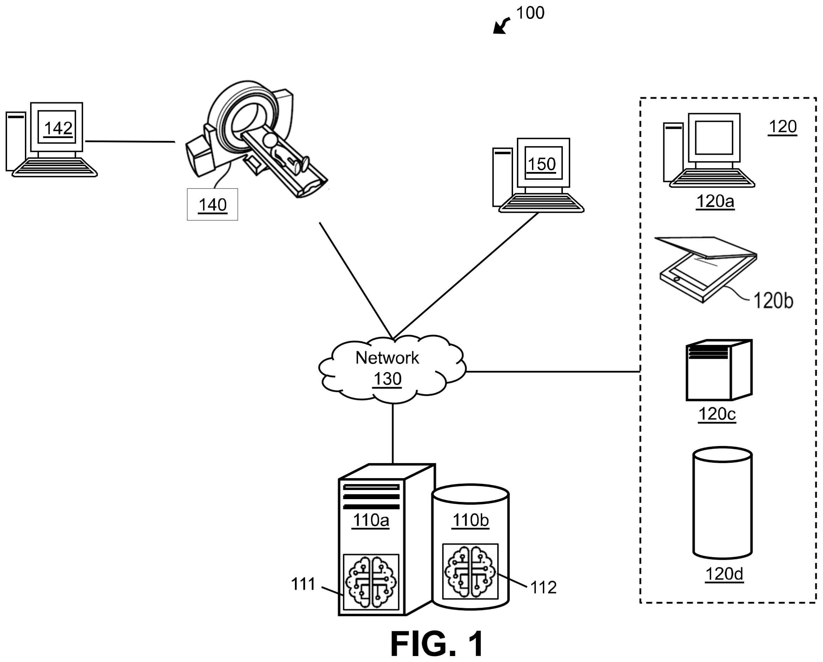

**FIG. 1** illustrates components of a multi-model radiotherapy planning validation system **100** (system **100),** according to an embodiment. The system **100** may include an analytics server **110a,** system database **110b,** validation model **111,** plan optimizer **112,** end-user devices **120a-d** (collectively end-user devices **120),** and an administrator computing device **150.** Various components depicted in **FIG. 1** may belong to a radiation therapy clinic at which patients may receive radiation therapy treatment, in some cases via one or more radiation therapy machines located within the clinic (e.g., medical device **140).** The above-mentioned components may be connected through a network **130.** Examples of the network **130** may include but are not limited to, private or public LAN, WLAN, MAN, WAN, and the Internet. The network **130** may include wired and/or wireless communications according to one or more standards and/or via one or more transport mediums.

The communication over the network **130** may be performed in accordance with various communication protocols such as Transmission Control Protocol and Internet Protocol (TCP/IP), User Datagram Protocol (UDP), and IEEE communication protocols. In one example, the network **130** may include wireless communications according to Bluetooth specification sets or another standard or proprietary wireless communication protocol. In another example, the network **130** may also include communications over a cellular network, including, e.g., a GSM (Global System for Mobile Communications), CDMA (Code Division Multiple Access), and EDGE (Enhanced Data for Global Evolution) network.

The system **100** is not confined to the components described herein and may include additional or other components, not shown for brevity, which is to be considered within the scope of the embodiments described herein.

The analytics server **110a** may generate and display an electronic platform configured to use various computer models **111-112** to identify attributes of an RTTP for a patient's treatment. The electronic platform may include a graphical user interface (GUI) displayed on the end-user devices **120** and/or the administrator computing device **150.** An example of the electronic platform generated and hosted by the analytics server **110a** may be a web-based application or a website configured to be displayed on different electronic devices, such as mobile devices, tablets, personal computers, and the like.

In a non-limiting example, a physician operating the physician device **120b** may access the platform, input patient attributes or characteristics and other data, and further instruct the analytics server **110a** to generate an RTTP for the patient. Additionally or alternatively, the analytics server **110a** may only optimize a particular attribute of the RTTP (e.g., optimize treatment beam angles).

The analytics server **110a** may recommend the RTTP (e.g., beam angles and other radiation parameters and/or treatment plan attributes) used for proton radiation, photon radiation, and/or electron radiation. In particular, analytics server **110a** may utilize the plan optimizer computer model **112** to generate the RTTP attributes. The plan optimizer computer model **112** may iterate through various possible RTTP attributes and evaluate each permutation until it converges upon a desired RTTP.

As discussed herein, the analytics server **110a** may also consider the output of the validation model **111** in order to control the functionality of the plan optimizer model **112.** That is, the analytics server **110a** may execute the validation model **111** in conjunction with the plan optimizer computer model **112** in order to create efficiencies in the execution of the plan optimizer computer model **112.** Further, the analytics server **110a** may transmit the beam angles and other radiation parameters and/or treatment plan attributes to one or more other servers. Additionally, or alternatively, the analytics server **110a** (another server) may adjust the configuration of one or more devices (e.g., the medical device **140)** based on the optimized beam angle. For instance, the RTTP may be directly sent to the medical device **140** and/or the computer **142** that functionally controls the medical device **140.**

The medical device **140** may be any medical device used in the radiation therapy treatment of a patient (such as a CT scan machine, radiation therapy machine (e.g., a linear accelerator, particle accelerator (including circular accelerators), or a cobalt machine)). The medical device **140** may also include an imaging device capable of emitting radiation such that the medical device **140** may perform imaging according to various methods to accurately image the internal structure of a patient. For instance, the medical device **140** may include a rotating system (e.g., a static or rotating multi-view system). A non-limiting example of a multi-view system may include stereo systems (e.g., two systems may be arranged orthogonally).

The analytics server **110a** may host a website accessible to users operating any of the electronic devices described herein (e.g., end users or medical professionals), where the content presented via the various webpages may be controlled based upon each particular user's role or viewing permissions. The analytics server **110a** may be any computing device comprising a processor and non-transitory machine-readable storage capable of executing the various tasks and processes described herein. The analytics server **110a** may employ various processors such as central processing units (CPU) and graphics processing unit (GPU), among others. Non-limiting examples of such computing devices may include workstation computers, laptop computers, server computers, and the like. While the system **100** includes a single analytics server **110a,** the analytics server **110a** may include any number of computing devices operating in a distributed computing environment, such as a cloud environment.

The analytics server **110a** may execute software applications configured to display the electronic platform (e.g., host a website), which may generate and serve various webpages to each end-user devices **120.** Different users may use the website to view and/or interact with the recommended (optimized) results. Different servers, such as a clinic server **120c** may also use the recommended results in downstream processing.

The analytics server **110a** may be configured to require user authentication based upon a set of user authorization credentials (e.g., username, password, biometrics, cryptographic certificate, and the like). The analytics server **110a** may access the system database **110b** configured to store user credentials, which the analytics server **110a** may be configured to reference in order to determine whether a set of entered credentials (purportedly authenticating the user) match an appropriate set of credentials that identify and authenticate the user.

The analytics server **110a** may generate and host webpages based upon a particular user's role within the system **100.** In such implementations, the user's role may be defined by data fields and input fields in user records stored in the system database **110b.** The analytics server **110a** may authenticate the user and may identify the user's role by executing an access directory protocol (e.g., LDAP). The analytics server **110a** may generate webpage content that is customized according to the user's role defined by the user record in the system database **110b.**

The analytics server **110a** may receive various clinical objectives, patient data, and treatment data from the end-user devices **120.** For instance, a physician may access the platform provided by the analytics server **110a** using a physician device **120b.** The physician may input various patient attributes and/or clinical objectives using one or more input elements of the platform. The analytics server **110a** may then execute various methods discussed herein and display an RTTP on the platform.

The end-user devices **120** may be any computing device comprising a processor and a non-transitory machine-readable storage medium capable of performing the various tasks and processes described herein. Non-limiting examples of end-user devices **120** may be a workstation computer, laptop computer, tablet computer, and server computer. In operation, various users may use end-user devices **120** to access the GUI operationally managed by the analytics server **110a.** Specifically, the end-user devices **120** may include clinic computer **120a,** physician device **120b,** clinic server **120c,** and clinic database **120d.**

In order to generate the RTTP, the analytics server **110a** may then execute various models (e.g., validation model **111** and/or a plan optimizer model **112)** to analyze the retrieved/received data.

The administrator computing device **150** may represent a computing device operated by a system administrator. The administrator computing device **150** may be configured to display beam angles, radiation parameters, and/or other radiation therapy treatment attributes generated by the analytics server **110a;** monitor the validation model **111** utilized by the analytics server **110a,** and/or end-user devices **120;** review feedback; and/or facilitate training or retraining (calibration) of the models **111-112** that are maintained by the analytics server **110a.**

The analytics server **110a** may be in communication (real-time or near real-time) with the medical device **140** (or its computer **142),** such that a server/computer hosting the medical device **140** can adjust the medical device **140** based on the RTTP (e.g., beam angles, treatment attributes and/or radiation parameters determined by the analytics server **110a).** For instance, the radiation therapy machine may adjust the gantry, beam blocking device (e.g. multileaf collimator MLC), and couch based on optimized beam angles, where the optimized beam angle is an angle of the medical device **140** that emits radiation in a direction of the PTVs. The analytics server **110a** may transmit instructions to the radiation therapy machines indicating any number or type of radiation parameters and/or treatment attributes to facilitate such adjustments.

The models **111** and/or **112** may represent any collection of algorithmic logic and/or artificial intelligence models (e.g., using various machine learning techniques). For instance, the validation model **111** may include various algorithms to monitor and evaluate the performance of the plan optimizer computer model **112.** As discussed herein, the analytics server **110a** may execute the models in conjunction with each other and then revise one or more configuration parameters of the plan optimizer **112.** Non-limiting examples of a configuration parameter revised by the analytics server **110a** may include a search space that is limited/revised using the attributes of the treatable sector. Another example may include stopping the plan optimizer computer modeling from further iterating through future permutations. As a result, the efficiencies discussed herein can be achieved without revising the plan optimizer computer model **112** itself. For instance, the validation model **111** can be executed independently and used in conjunction with a clinic's existing plan optimizer. Therefore, the plan optimizer (or the system infrastructure) can be retrofitted using the methods and systems discussed herein. This minimal interference with existing infrastructure allows for the improvement of a plan optimizer without the need to revise its source code.

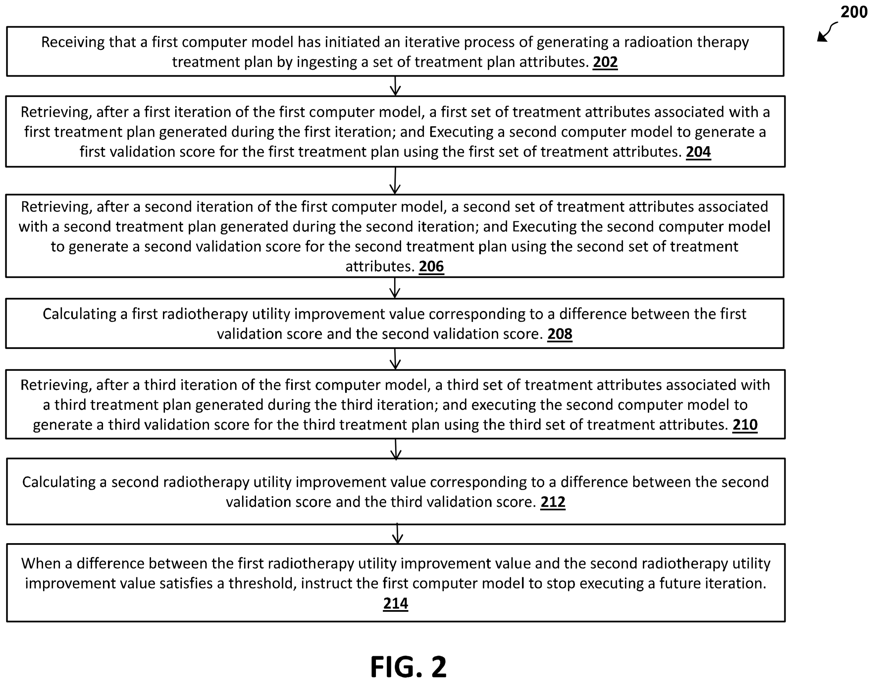

**FIG. 2** illustrates a flow diagram of a process executed in a multi-model radiotherapy planning validation system, according to an embodiment. The method **200** includes steps **202-214.** However, other embodiments may include additional or alternative steps or may omit one or more steps altogether. One or more steps of method **200** may be executed by any number of computing devices operating in the distributed computing system described in **FIG. 1****.** For instance, one or more computing devices may locally perform part, or all of the steps described in **FIG. 2** or a cloud device may perform such steps. In the following discussion, steps performed by the analytics server may in general be performed by the at least one processor.

At step **202,** the analytics server may receive an indication that a first computer model has initiated an iterative process of generating a radiation therapy treatment plan by ingesting a set of treatment plan attributes.

In some embodiments, the analytics server may receive an indication that a first computer model has commenced an iterative process to generate an RTTP. A non-limiting example of the first computer model may be the plan optimizer computer model (e.g., model **112** discussed in **FIG. 1****).** This initiation may be signaled by the ingestion of a predefined set of treatment plan attributes, which is the beginning step for tailoring the therapy to individual patient needs. In some embodiments, the analytics server may receive the indication from the first computer model itself. For instance, the first computer model may ingest patient data (e.g., the patient's anatomical data, tumor size, and/or location, desired radiation dose levels, and/or specific constraints related to organs at risk) and start the iterative process of generating the RTTP. After a defined number of iterations and/or time, the first computer model may then transmit a signal to the analytics server indicating that the first computer model has reached a predefined point, for example a predefined point in the iterative process.

The receipt of the indication that the first computer model has started the iterative process may be used to trigger the execution of the method **200.** That is, the plan optimizer computer model starting to iterate through different permutations may trigger the optimization cycle within the analytics server. The indication not only indicates that the first computer model is ready to proceed (or has already started its iterations) but may also confirm that all preliminary data have been successfully ingested by the first computer model and validated for processing.

Upon receipt of this signal, the analytics server may log the event, thus maintaining a record of each optimization/iteration attempt. This logging and auditing may be used to refine the first computer model's performance over time. Furthermore, this initiation phase may allow the system to ensure that all systems are operational, and that the data integrity is maintained before the optimization algorithm actively begins manipulating the radiation therapy parameters.

At step **204,** the analytics server may retrieve, after a first iteration of the first computer model, a first set of treatment attributes associated with a first treatment plan generated during the first iteration. The analytics server may also execute a second computer model to generate a first validation score for the first treatment plan using the first set of treatment attributes.

Following a defined number of iterations and/or a defined amount of time after the first computer model has started its iterative process (e.g., iterations of the radiation therapy treatment plan generation by the first computer model), the analytics server may then retrieve a set of treatment attributes associated with the newly generated (or the latest) RTTP. This retrieval process may be executed by the analytics server, which may systematically access and compile the data, delineating various critical parameters of the RTTP. The parameters retrieved may typically include the distribution and/or intensity of radiation doses planned across different treatment fields, the spatial arrangement and/or angles of radiation beams, and/or any modulations specific to the treatment session. This set of attributes may form the foundational data upon which subsequent evaluations and optimizations are based, ensuring that each iteration refines these attributes closer to optimal therapeutic efficacy.

The process of retrieving these treatment attributes may be used to maintain the integrity and progression of the optimization process by the first computer model. By systematically capturing detailed treatment data after each iteration (or a defined number of iterations and/or a defined amount of time), the analytics server can provide a comprehensive dataset that allows for precise assessment and necessary adjustments in subsequent iterations.

In some embodiments, the retrieval may not be merely a collection of data but a dynamic input into the ongoing iterative process that aims to converge on the most effective or optimized RTTP.

Upon the successful retrieval of the first set of treatment attributes from the initial iteration, the analytics server may evaluate the retrieved data. In this phase, the analytics server may execute a second computer model (e.g., the validation computer model) designed specifically to assess the clinical viability of the treatment plan that has been generated at this stage by the first computer model.

This second computer model may apply various protocols and defined rules (e.g., templates) to generate a first validation score based on the ingested treatment attributes, such as dose distribution, beam angles, and/or intensity patterns. This validation score may be a quantitative assessment that measures how well the proposed treatment plan meets predefined clinical criteria and safety thresholds, which are crucial for patient-specific treatment efficacy and safety.

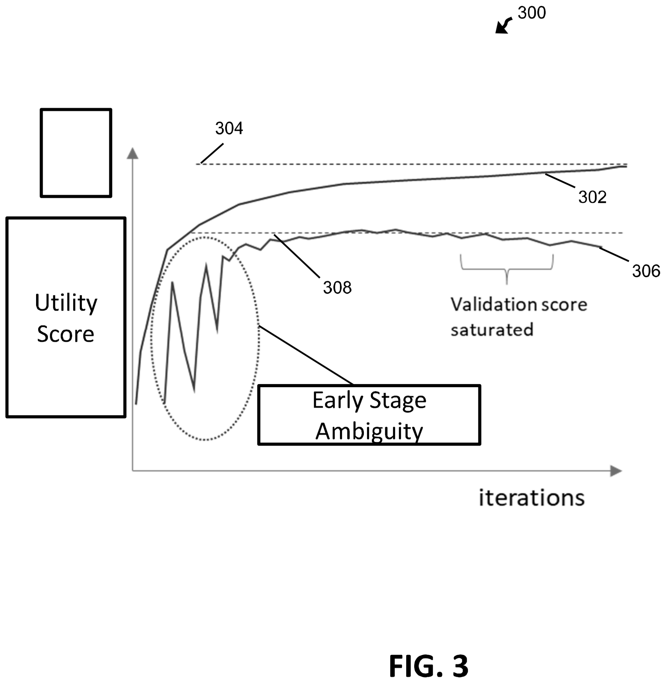

The execution of the second computer model by the analytics server may integrate empirical data with predictive analytics to ensure the treatment plan's feasibility. The validation score produced may serve as a decisive metric that informs whether the treatment plan, as configured by the first defined set of iterations or time (such as a first defined amount of time), aligns with optimal therapeutic outcomes and adheres to stringent clinical guidelines. The analytics server may utilize this score to determine necessary adjustments or to validate the continuation of the optimization process, thereby embedding a systematic review mechanism that enhances the overall quality and safety of the radiation therapy planning. In some embodiments, the validation score may differ from the traditional cost value used by plan optimizers. For instance, the validation score may not necessarily correspond to the utility score, as will be discussed with respect to **FIG. 3****.**

Though aspects of the embodiment discussed herein refer to a "first" iteration, the method 200 can be implemented using a defined number of iterations, a defined amount of time, defined amount of computing resources used, or any other defined parameter. For instance, the score may be generated after 20 iterations or after 5 minutes after the plan optimizer computer model has started its iterative process. The method **200** may generally evaluate the RTTP after a defined milestone or iteration progress metric (time or number of iterations) and use the validation score to evaluate the progress of the plan optimizer.

As used herein, a validation score may refer to a quantitative metric calculated by e.g. the second computer model to assess the clinical viability and/or safety of a radiation therapy treatment plan. Therefore, the validation score identifies how well the plan meets predefined clinical criteria and safety thresholds, ensuring that the treatment is both effective for the patient and adheres to medical standards. The analytics server may use various protocols to calculate the validation score, such as any combination of one or more of: DVH Compliance, tumor control probability, normal tissue compliance probability, normal tissue complications probability, conformity index, homogeneity index, and/or the like. In some embodiments, the validation score may correspond to whether the RTTP is within clinical bounds or treatment objectives. For instance, the validation score may indicate (or otherwise penalize) whether the RTTP would result in any combination of one or more of: a violation of an organ at risk dose limit, a beam angle constraint, a hot spot attribute, a cold spot attribute, or a planning target volume dose limit.

In general, the validation score may be a function based on an independent model that describes the value of a certain RTTP for a given patient. In some embodiments, the validation score may mirror or otherwise correspond to a utility score or an optimizer utility function, which is sometimes used as a cost function by plan optimizers. However, unlike the utility function, the validation score may not be used in the solution space search. Instead, the validation score generated by the second computer model may be used to determine the termination of the optimization at the point when the optimal solution is no longer improving, or is no longer improving by a threshold amount.

At step **206,** the analytics server may retrieve, after a second iteration of the first computer model, a second set of treatment attributes associated with a second treatment plan generated during the second iteration. The analytics server may also execute the second computer model to generate a second validation score for the second treatment plan using the second set of treatment attributes.

Following a second defined number of iterations or defined amount of time (after the step **204),** the analytics server may collect a second set of treatment attributes from the treatment plan generated during this latest iteration. These attributes may also encompass comprehensive details, such as updated dose distributions, modifications to beam angles, and any changes in the modulation or configuration of the radiation beams used in the treatment plan. The retrieval of these attributes may be used to maintain the continuity and relevance of the treatment optimization process and to ensure that each iteration builds upon the accuracy and efficacy of the previous one (e.g., the first set of collected data).

The analytics server may gauge or measure the iterative improvements and adaptations made by the first computer model by comparing how the first computer model is iteratively converging upon the ultimate RTTP. By systematically extracting and compiling these updated treatment attributes, the analytics server may ensure that the data driving the optimization process is the most current and reflective of the best possible treatment strategy. This continual update and retrieval process not only facilitates a dynamic optimization loop but also ensures that each subsequent evaluation of the treatment plan, using the second computer model, is based on the latest and most accurate information.

After retrieving the second set of treatment attributes from the updated treatment plan, the analytics server may again execute the second computer model to generate a second score associated with the latest treatment plan. Accordingly, the analytics server may gauge or measure the enhancements or modifications made in the second iteration of the treatment planning process. Using the updated attributes, the second computer model may calculate a second validation score. This score may evaluate the revised treatment plan against the same rigorous clinical criteria and safety thresholds as the first validation. The score may provide a quantitative assessment that indicates whether the adjustments made after the first iteration (step **204)** have moved the treatment plan closer to the ideal therapeutic outcome.

The execution of the second computer model by the analytics server may be used for continuous/periodic monitoring of the improvement in the treatment planning process. Accordingly, the validation score may serve as a feedback mechanism, informing the analytics server and medical team of the effectiveness and safety of the adjustments made to the treatment plan by the first computer model. By systematically applying the second computer model to generate validation scores after each iteration, the process ensures that each version of the treatment plan not only strives for higher clinical efficacy but also adheres strictly to safety standards, thus optimizing patient care in a precise and informed manner.

Though aspects of the embodiment discussed herein discuss the "second" iteration, the method **200** can be implemented using a defined number of iterations, a defined amount of time, a defined amount of computing resources used, or any other defined method. For instance, the score may be generated after 20 iterations or after 5 minutes after the plan optimizer computer model has started its iterative process. The method **200** may generally evaluate the RTTP after a defined milestone or iteration progress metric (time or number of iterations) and use the score to evaluate the progress of the plan optimizer.

At step **208,** the analytics server may calculate a first radiotherapy utility improvement value corresponding to a difference between the first validation score and the second validation score.

Upon generating the first and second validation scores for the successive sets of iterations of the treatment plans generated by the first computer model, the analytics server may calculate a first radiotherapy utility improvement value to measure the progress of the treatment optimization. The calculation of the first radiotherapy utility improvement value may represent the difference between the first and second validation scores (calculated in steps **204** and 206). This metric may quantitatively reflect the degree of improvement or regression in the treatment plan's clinical viability and safety from one iteration to the next. By quantifying the improvement, the analytics server can assess whether the changes implemented have resulted in a statistically significant enhancement of the treatment's potential success or if further adjustments are necessary. This calculated value may be used to guide the decision-making process, indicating whether to continue refining the treatment plan or to finalize it if the desired therapeutic efficacy and safety thresholds are met. The use of this metric may ensure a data-driven approach to radiation therapy planning, aiming for continuous improvement in treatment outcomes.

At step **210,** the analytics server may retrieve, after a third iteration of the first computer model, a third set of treatment attributes associated with a third treatment plan generated during the third iteration. The analytics server may also execute the second computer model to generate a third validation score for the third treatment plan using the third set of treatment attributes.

Similar to the steps of **204** and **206,** the analytics server may collect the latest RTTP generated by the first computer model after a defined number of iterations and/or time. After retrieving the third RTTP, the analytics server may then execute the second computer model to generate a third validation score for the third treatment plan.

At step **212,** the analytics server may calculate a second radiotherapy utility improvement value corresponding to a difference between the second validation score and the third validation score.

After obtaining the second and third validation scores, the analytics server may execute the protocol (similar to the one performed in the step **208)** to quantify the improvements or changes between these iterations of the treatment plan. This may involve computing the second radiotherapy utility improvement value, which measures the difference between the second and third validation scores. This calculated difference may serve as an indicator of whether the adjustments made in the latest iteration, for example in the third iteration of the first computer model, have enhanced the treatment plan's effectiveness and safety or if they require further refinement.

The process of calculating the second radiotherapy utility improvement value may be used for assessing the progressive optimization of the treatment plan. It provides a quantitative measure that helps the medical team determine the efficacy of the iterative changes made based on the second computer model's feedback. By systematically quantifying the improvements, the analytics server facilitates informed decision-making regarding the continuation of the optimization process. This step ensures that each iteration contributes meaningfully towards achieving an optimal treatment plan, with each calculation acting as a checkpoint to validate the direction and impact of the changes implemented.

At step **214,** the analytics server may, when a difference between the first radiotherapy utility improvement value and the second radiotherapy utility improvement value satisfies a threshold, instruct the first computer model to stop executing a future iteration, or to stop the iterative process.

Once the analytics server calculates the first and second radiotherapy utility improvement values, it then assesses these values against a predetermined threshold. If the difference between the two improvement values meets or exceeds this threshold, it indicates that the adjustments made between the iterations have either achieved the desired optimization goal or that further iterations are unlikely to result in significant improvement.

Upon this determination, the analytics server may instruct the first computer model to cease further iterations. This command may be issued to prevent unnecessary computational effort and resource utilization when the treatment plan has reached a point of diminishing returns in terms of clinical improvement. Stopping the process at this stage ensures that the treatment planning remains efficient, cost-effective, and aligned with achieving the best possible patient outcomes without overextending the optimization cycle and/or overusing any computing resources.

The stoppage criteria or the iteration stop criteria (e.g., the threshold) may be selected by the medical professional or pre-programmed in accordance with clinical standards and/or clinic-specific rules. The threshold may be strategically defined to ensure that the optimization doesn't end prematurely, especially during the early stages of the process. For instance, initially, the utility function, which assesses the effectiveness of the treatment, might show rapid changes as the first computer model generates different RTTPs. During this phase, variations in the validation score (e.g., depicted in **FIG. 3** as early ambiguity), which evaluates safety and clinical viability, might not provide a reliable indication of the final plan's quality. Stopping the optimization based on these early fluctuations could lead to suboptimal treatment plans that may not be clinically viable.

Furthermore, as the optimization progresses, the validation score might show some irregular patterns or noise, indicating that the treatment plan is still stabilizing. Relying on a single instance where the validation score meets the stopping criterion might be misleading due to this noise. To handle this effectively, a longer observation period may be used by the analytics server to confirm that any improvements in the treatment plan are consistent and stable. One practical method to establish a robust stopping point is to monitor a sliding average of the validation scores from recent iterations, such as the last five. If this average falls by a certain percentage, such as 5% from the highest observed validation score, it suggests that additional iterations are unlikely to significantly enhance the plan. This approach helps ensure that the optimization process concludes at the most appropriate time, securing an optimal balance between the treatment's effectiveness and safety.

The RTTP generation and optimization process can be visualized through a convergence curve that aims to maximize the user-defined utility based on specific patient cases. Referring now to **FIG. 3****,** a chart **300** provides a visual representation of the solution score and different iterations used by the first computer model.

In the depicted embodiment, the utility of an RTTP may be defined by evaluating how the mean radiation dose to the parotid gland affects the probability of xerostomia and its subsequent impact on the patient's well-being. The shape of this utility's convergence curve, which might typically show a smooth and monotonically increasing function, is contingent on the chosen optimization strategy, such as a gradient-search method. This curve is expected to reach a saturation point, where further iterations no longer yield significant improvements, particularly as the optimization explores increasingly complex solutions, like adding more control points. In the chart **300,** the Y-Axis represents the utility score of each RTTP. The X-axis represents the number of iterations by the first computer model. As illustrated by line **302,** the utility score increases until it plateaus near a line **304.**

However, the decision to end the optimization process is not solely based on the saturation of the utility function (e.g., whether the score has plateaued near the line **304).** Instead, the stoppage criteria or iteration stop criteria depend on the validation score.

The validation score, depicted as line **306,** may be derived from an independent model and may align with or differ from the utility function's principles. For instance, while the utility function might directly relate to clinical outcomes and patient preferences, the validation score could be based on different modeling principles, such as DVH estimations.

The critical point occurs when the validation score reaches saturation line **308,** often earlier than the utility function. This indicates that additional increases in the utility score are unlikely to translate into real and tangible improvements in patient outcomes, suggesting that the treatment plan is optimized to the extent that it no longer significantly enhances the likelihood of curing the patient without adverse effects. The methods and systems discussed herein ensure that the optimization performed by the first computer model is stopped at the most effective treatment plan, avoiding unnecessary complications from over-optimization.

Accordingly, the validation score is different than the cost function used by the plan optimizer (first computer model). For instance, the set of criteria evaluated in order to generate the validation score may be different than those evaluated by the first computer model to evaluate the RTTP. In some embodiments, the set of criteria evaluated by the second computer model to generate the validation score may include clinical guidelines, historical treatment outcomes, or predictive models for side effects. For example, the set of criteria evaluated in order to generate the validation score may be different from the cost function used to evaluate and optimize the RTTP.

In the process of generating and optimizing the RTTP, once the criteria to stop further iterations are met and the first computer model is instructed to cease iterating, deciding on the final RTTP can be performed using various protocols. In a first non-limiting example, the analytics server might choose the most recent version of the RTTP, assuming the last iteration before stopping was the most refined. In a second non-limiting example, the analytics server may select an RTTP from an earlier iteration. For example, the analytics server may select the RTTP before the differences between the validation scores start having a negative trend. For example, the analytics server may select the RTTP before a start of any deterioration in successive validation scores. In a third non-limiting example, the analytics server may select the RTTP that received the highest validation score throughout all the iterations, indicating it was the best according to the assessment criteria used.

In some embodiments, if the utility improvement value is below a threshold, the analytics server may instruct the first computer model to restart its iterations. For instance, if the RTTP does not indicate an increase in validation score but is still below a certain utility score, the first computer model may be in a loop where the RTTP is not improving. As a result, the analytics server may stop the iterations, instruct the first computer model to change one or more attributes of the RTTP, then restart its iterative process of optimization.

In some embodiments, a single machine learning model may bifurcate its operations and use the bi-model paradigm discussed herein to act as both models. For instance, the machine learning model may use adversarial techniques to both generate the RTTP and validate its content separately.

In some embodiments, one or more of the thresholds discussed herein may be dynamically adjusted or pre-defined in accordance with a particular patient's attributes. For instance, a medical professional may adjust one or more thresholds discussed herein for a particular patient in accordance with the patient's diagnosis (e.g., tumor size or location). In some embodiments, the algorithm used to calculate the validation score generated for one patient may be different than the algorithm used for other patients.

### Non-limiting Example:

A medical team of a clinic may use the systems and methods discussed herein to generate an RTTP for a patient with lung cancer. Initially, a medical physicist inputs patient-specific data into the system, such as the tumor size, location, and proximity to critical organs. The plan optimizer, leveraging these attributes, begins generating the RTTP. After a defined number of iterations (e.g., ten iterations in this non-limiting example or a defined number of minutes), and as a preliminary RTTP is generated, the analytics server retrieves the treatment attributes of this preliminary RTTP and uses a second model to calculate a validation score, assessing the preliminary RTTP's safety and effectiveness.

After a defined number of iterations (e.g., 20 iterations or after 3 minutes), the analytics server repeats the process by retrieving the secondary RTTP generated and generating a second validation score for the secondary RTTP. If necessary, this process might be repeated after a defined number of iterations or times when the second computer model is used to generate a third validation score.

The analytics server then assesses whether further adjustments and the continuation of the iterations by the plan optimizer are yielding significant improvements that are more than a threshold. This is achieved by determining whether the validation score is increasing or decreasing, and, if so, whether the amount of decrease is below or higher than a threshold. If the differences in improvement values between iterations fall below a predetermined clinical significance threshold-indicating that further iterations are unlikely to result in substantial benefits-the analytics server instructs the plan optimizer to cease further iterations. At this point, the plan optimizer is instructed to finalize the RTTP and output the finalized RTTP as the finalized treatment plan so that the medical team can review its accuracy and efficacy. This finalized treatment plan, optimized for both efficacy and safety within the clinic's set clinical and technical boundaries, is then reviewed and approved by the oncologist.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of this disclosure or the claims.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

The actual software code or specialized control hardware used to implement these systems and methods is not limiting of the claimed features or this disclosure. Thus, the operation and behavior of the systems and methods were described without reference to the specific software code being understood that software and control hardware can be designed to implement the systems and methods based on the description herein.

When implemented in software, the functions may be stored as one or more instructions or code on a non-transitory computer-readable or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the embodiments described herein and variations thereof. Various modifications to these embodiments will be readily apparent to those skilled in the art, and the principles defined herein may be applied to other embodiments without departing from the scope of the subject matter disclosed herein. Thus, the present disclosure is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope being indicated by the following claims.
**The following numbered clauses are subject-matter of, but not currently claims of, the present application.**

### CLAUSES

1. A system comprising a computer-readable medium having non-transitory instructions, that when executed, cause a processor to:
   receive an indication that a first computer model has initiated an iterative process of generating a radiation therapy treatment plan by ingesting a set of treatment plan attributes;
   retrieve, after a first iteration of the first computer model, a first set of treatment attributes associated with a first treatment plan generated during the first iteration;
   execute a second computer model to generate a first validation score for the first treatment plan using the first set of treatment attributes;
   retrieve, after a second iteration of the first computer model, a second set of treatment attributes associated with a second treatment plan generated during the second iteration;
   execute the second computer model to generate a second validation score for the second treatment plan using the second set of treatment attributes;
   calculate a first radiotherapy utility improvement value corresponding to a difference between the first validation score and the second validation score;
   retrieve, after a third iteration of the first computer model, a third set of treatment attributes associated with a third treatment plan generated during the third iteration;
   execute the second computer model to generate a third validation score for the third treatment plan using the third set of treatment attributes;
   calculate a second radiotherapy utility improvement value corresponding to a difference between the second validation score and the third validation score; and
   when a difference between the first radiotherapy utility improvement value and the second radiotherapy utility improvement value satisfies a threshold, instruct the first computer model to stop executing a future iteration.
2. The system of clause 1, wherein the validation score corresponds to whether a treatment plan is within a clinical boundary.
3. The system of clause 2, wherein the clinical boundary corresponds to an organ at risk dose limit, a beam angle constraint, a hot spot attribute, a cold spot attribute, or a planning target volume dose limit.
4. The system of any of clause 1 to clause 3, wherein the second computer model is configured to generate the validation score based on a different set of criteria than those used by the first computer model, the set of criteria comprising clinical guidelines, historical treatment outcomes, or predictive models for side effects.
5. The system of any of clause 1 to clause 4, wherein the instructions further cause the processor to: adjust the threshold used to determine when to stop the first computer model's iterative process based on a patient-specific risk profile or physician input.
6. The system of any of clause 1 to clause 5, wherein the threshold is dynamically adjusted during the iterative process based on real-time analysis of a rate of change in any of the validation scores.
7. The system of any of clause 1 to clause 6, wherein the instructions further cause the processor to: output the third set of treatment attributes associated with a third treatment plan as a final result of execution of the first computer model.
8. A system comprising:
   a first computer model configured to generate a radiation therapy treatment plan by ingesting a set of treatment plan attributes;
   a second computer model configured to validate the first computer model; and
   a processor in communication with the first computer model and the second computer model, the processor configured to:
      receive an indication that the first computer model has initiated an iterative process of generating the radiation therapy treatment plan by ingesting the set of treatment plan attributes;
      retrieve, after a first iteration of the first computer model, a first set of treatment attributes associated with a first treatment plan generated during the first iteration;
      execute the second computer model to generate a first validation score for the first treatment plan using the first set of treatment attributes;
      retrieve, after a second iteration of the first computer model, a second set of treatment attributes associated with a second treatment plan generated during the second iteration;
      execute the second computer model to generate a second validation score for the second treatment plan using the second set of treatment attributes;
      calculate a first radiotherapy utility improvement value corresponding to a difference between the first validation score and the second validation score;
      retrieve, after a third iteration of the first computer model, a third set of treatment attributes associated with a third treatment plan generated during the third iteration;
      execute the second computer model to generate a third validation score for the third treatment plan using the third set of treatment attributes;
      calculate a second radiotherapy utility improvement value corresponding to a difference between the second validation score and the third validation score; and
      when a difference between the first radiotherapy utility improvement value and the second radiotherapy utility improvement value satisfies a threshold, instruct the first computer model to stop executing a future iteration.
9. The system of clause 8, wherein the validation score corresponds to whether a treatment plan is within a clinical boundary.
10. The system of clause 9, wherein the clinical boundary corresponds to an organ at risk dose limit, a beam angle constraint, a hot spot attribute, a cold spot attribute, or a planning target volume dose limit.
11. The system of any of clause 8 to clause 10, wherein the second computer model is configured to generate the validation score based on a different set of criteria than those used by the first computer model, the set of criteria comprising clinical guidelines, historical treatment outcomes, or predictive models for side effects.
12. The system of any of clause 8 to clause 11, wherein processor is further configured to adjust the threshold used to determine when to stop the first computer model's iterative process based on a patient-specific risk profile or physician input.
13. The system of any of clause 8 to clause 12, wherein the threshold is dynamically adjusted during the iterative process based on real-time analysis of a rate of change in any of the validation scores.
14. The system of any of clause 8 to clause 13, wherein the processor is further configured to output the third set of treatment attributes associated with a third treatment plan as a final result of execution of the first computer model.

## Claims

1. A method comprising:
receiving, by at least one processor, an indication that a first computer model has initiated an iterative process of generating a radiation therapy treatment plan by ingesting a set of treatment plan attributes;
retrieving, by at least one processor after a first iteration of the first computer model, a first set of treatment attributes associated with a first treatment plan generated during the first iteration;
executing, by the at least one processor, a second computer model to generate a first validation score for the first treatment plan using the first set of treatment attributes;
retrieving, by at least one processor after a second iteration of the first computer model, a second set of treatment attributes associated with a second treatment plan generated during the second iteration;
executing, by the at least one processor, the second computer model to generate a second validation score for the second treatment plan using the second set of treatment attributes;
calculating, by the at least one processor, a first radiotherapy utility improvement value corresponding to a difference between the first validation score and the second validation score;
retrieving, by at least one processor after a third iteration of the first computer model, a third set of treatment attributes associated with a third treatment plan generated during the third iteration;
executing, by the at least one processor, the second computer model to generate a third validation score for the third treatment plan using the third set of treatment attributes;
calculating, by the at least one processor, a second radiotherapy utility improvement value corresponding to a difference between the second validation score and the third validation score; and
when a difference between the first radiotherapy utility improvement value and the second radiotherapy utility improvement value satisfies a threshold,
instructing, by the at least one processor, the first computer model to stop executing a future iteration.

2. The method of claim 1, wherein the validation score corresponds to whether a treatment plan is within a clinical boundary.

3. The method of claim 2, wherein the clinical boundary corresponds to an organ at risk dose limit, a beam angle constraint, a hot spot attribute, a cold spot attribute, or a planning target volume dose limit.

4. The method of any preceding claim, wherein the second computer model is configured to generate the validation score based on a different set of criteria than those used by the first computer model, the set of criteria comprising clinical guidelines, historical treatment outcomes, or predictive models for side effects.

5. The method of any preceding claim, further comprising:
adjusting, by at least one processor, the threshold used to determine when to stop the first computer model's iterative process based on a patient-specific risk profile or physician input.

6. The method of any preceding claim, wherein the threshold is dynamically adjusted during the iterative process based on real-time analysis of a rate of change in any of the validation scores.

7. The method of any preceding claim, further comprising:
outputting, by the at least one processor, the third set of treatment attributes associated with a third treatment plan as a final result of execution of the first computer model.

8. A computer system configured to perform a method comprising:
receive an indication that a first computer model has initiated an iterative process of generating a radiation therapy treatment plan by ingesting a set of treatment plan attributes;
retrieve, after a first iteration of the first computer model, a first set of treatment attributes associated with a first treatment plan generated during the first iteration;
execute a second computer model to generate a first validation score for the first treatment plan using the first set of treatment attributes;
retrieve, after a second iteration of the first computer model, a second set of treatment attributes associated with a second treatment plan generated during the second iteration;
execute the second computer model to generate a second validation score for the second treatment plan using the second set of treatment attributes;
calculate a first radiotherapy utility improvement value corresponding to a difference between the first validation score and the second validation score;
retrieve, after a third iteration of the first computer model, a third set of treatment attributes associated with a third treatment plan generated during the third iteration;
execute the second computer model to generate a third validation score for the third treatment plan using the third set of treatment attributes;
calculate a second radiotherapy utility improvement value corresponding to a difference between the second validation score and the third validation score; and
when a difference between the first radiotherapy utility improvement value and the second radiotherapy utility improvement value satisfies a threshold, instruct the first computer model to stop executing a future iteration.

9. The system of claim 8, wherein the validation score corresponds to whether a treatment plan is within a clinical boundary.

10. The system of claim 9, wherein the clinical boundary corresponds to an organ at risk dose limit, a beam angle constraint, a hot spot attribute, a cold spot attribute, or a planning target volume dose limit.

11. The system of any of claim 8 to claim 10, wherein the second computer model is configured to generate the validation score based on a different set of criteria than those used by the first computer model, the set of criteria comprising clinical guidelines, historical treatment outcomes, or predictive models for side effects.

12. The system of any of claim 8 to claim 11, wherein the method further comprises:
adjusting the threshold used to determine when to stop the first computer model's iterative process based on a patient-specific risk profile or physician input.

13. The system of any of claim 8 to claim 12, wherein the threshold is dynamically adjusted during the iterative process based on real-time analysis of a rate of change in any of the validation scores;
and/or:
wherein the method further comprises:
outputting the third set of treatment attributes associated with a third treatment plan as a final result of execution of the first computer model.

14. The system of any of claim 8 to claim 13, comprising a computer-readable medium having non-transitory instructions, that when executed, cause the processor to perform the method.

15. The system of any of claim 8 to claim 13, comprising:
the first computer model configured to generate the radiation therapy treatment plan by ingesting the set of treatment plan attributes;
the second computer model configured to validate the first computer model; and
the processor in communication with the first computer model and the second computer model.
